# EUROPÄISCHE PATENTSCHRIFT

(11) **EP 2 193 820 B1**
(45) Veröffentlichungstag und Bekanntmachung des Hinweises auf die Patenterteilung: **20.11.2019**
(21) Anmeldenummer: 09177100.6
(22) Anmeldetag: 25.11.2009
(51) Int. Cl.: A61M 25/10, A61N 5/10, A61B 5/06

(54) **Applikator mit Ballonkatheter**
Applicator with balloon catheter
Applicateur avec un cathéter à ballonnet

(30) Priorität: 02.12.2008 DE 102008060162
(43) Veröffentlichungstag der Anmeldung: 09.06.2010
(73) Patentinhaber: Carl Zeiss Meditec AG, 07745 Jena (DE)
(72) Erfinder: Röder, Norman, 73430 Aalen (DE); Benker, Matthias, 89522 Heidenheim (DE)
(74) Vertreter: Müller & Schubert

(56) Entgegenhaltungen:
- EP-B- 0 850 660
- WO-A-2008/077073
- US-A- 5 447 497
- US-A1- 2008 140 001

## Beschreibung

Die vorliegende Erfindung betrifft einen Applikator mit Ballonkatheter gemäß dem unabhängigen Patentanspruch 1.

In der invasiven Strahlentherapie von Tumoren existieren verschiedene Methoden, um den Tumor, oder nach erfolgter Lumpektomie das Tumorbett, zu bestrahlen. Hierfür muss ein Zugang zum Tumor oder in das verbliebene Tumorbett geschaffen werden. Dieser zeitlich begrenzte Zugang kann über Ballonkatheter ermöglicht werden, welche speziell im Fall der Tumorbettbestrahlung als eine Art Platzhalter fungieren.

In den häufigsten Fällen wird der zusammengefaltete Ballonkatheter über einen dafür geschaffenen Biopsiekanal gelegt. Anschließend wird der Ballon mit einem Medium befüllt, so dass er seine definierte Form annimmt. Ein solcher Ballonkatheter ist beispielsweise in der US 5,621,780 A offenbart.

Als Füllmedium wird häufig eine isotonische Kochsalzlösung genutzt. Einmal um bei einer Ballonruptur das Patientenrisiko durch das unter Druck stehende Füllmedium zu minimieren, da sich Wasser kaum komprimieren lässt. Weiterhin lässt sich durch eine Kochsalzlösung besser als beispielsweise bei Gasen die Formstabilität des Ballons gewährleisten.

Neben dem Zugang zum Tumorbett hat der Ballonkatheter die Aufgabe, dass forminstabile Tumorbett in eine definierte Form zu bringen, vorzugsweise in die einer Kugel. Aber auch andere Geometrien sind möglich. So wird ohne komplexe Bestrahlungsplanung eine gleichmäßige Bestrahlung des umliegenden Gewebes ermöglicht.

Bei dieser Methode ist es für die Bestrahlungsplanung wichtig zu wissen, ob die tatsächliche Form des Ballons auch wirklich die gewünschte beziehungsweise erforderliche ist. Speziell bei Ballonen, die aus dehnbarem Material bestehen, kann die Form durch lokale Gegebenheiten von der definierten Grundform abweichen.

Eine Erhöhung des Innendrucks durch weitere Medienzuführung bietet hier keine Lösung, da sich der Ballon nur weiter ausdehnen würde, wobei das Risiko einer Ballonruptur steigt.

Wird eine Formabweichung nicht bemerkt, kommt es durch die unterschiedlichen Abstände zur Fehlbestrahlung des umliegenden Gewebes.

Ideal wäre hier der Einsatz von "festen Applikatoren". Der hierbei verwendeter fester Formkörper verdrängt ideal das umliegende Gewebe und ermöglicht so ein aufmodellieren des Gewebes um den Applikator. Feste Applikatoren garantieren eine stabile Anordnung während der Bestrahlung. Jedoch eignen sie sich aufgrund ihrer Haupteigenschaft nicht zum Einsatz für die fraktionierte Bestrahlung, da sie nach der Bestrahlung nicht minimal invasiv entfernt werden können.

Zur Sicherstellung der Ballonform wird in der Praxis mit verschiedenen Bild gebenden Verfahren die Ballonform nach der Platzierung im Körper überprüft. Nach der Bestrahlung, welche sich über mehrere Bestrahlungssitzungen erstrecken kann, wird das Füllmedium wieder abgesaugt und der Ballonkatheter durch den Biopsiekanal entfernt.

Techniken wie Computertomographie (CT) oder Ultraschall werden sehr häufig für die Visualisierung von Ballonkatheter im Patienten genutzt. Dabei wird jedoch die Bildgebung durch spezielle Eigenschaften des Ballonkatheters erschwert. Einmal werden die Ballons zur Formgebung meist mit einer wässrigen Lösung gefüllt, wodurch sich der Ballon kaum vom umgebenen Gewebe unterscheidet. Dies erschwert die Bildgebung durch CT oder Ultraschall. Luft würde sich zur Visualisierung sehr gut eignen. Aufgrund der hohen Kompression, die für die Formstabilität benötigt wird, ist dies jedoch mit einem erhöhten Risiko für den Patienten verbunden. Weiter müssen bei CT für die Sichtbarkeit des Ballonkatheters Röntgenstrahlen absorbierende Materialien beziehungsweise Strukturen in oder auf der Ballonwandung verwendet werden. Es besteht auch die Möglichkeit den Ballon mit einem Kontrastmittel oder Wasser Kontrastmittel Gemisch zu befüllen.

Daraus ergibt sich neben der prinzipbedingten Strahlenbelastung durch CT ein weiterer unerwünschter Nebeneffekt für den Patienten. Für die Bildgebung ist es notwendig, dass die Röntgenstrahlen absorbiert werden. Das bedeutet aber auch, dass ein Teil der Röntgenstrahlung während der Therapie absorbiert wird. Zur Kompensation muss entweder die Leistung der Röntgenquelle oder die Bestrahlungszeit erhöht werden.

Eine Lösung des Problems könnte die Änderung des Behandlungsablaufes sein. So könnte zum Beispiel der Ballon zuerst mit Luft oder Kontrastmittel befüllt werden. Nach der Lage und Formüberprüfung wird dieses Medium durch das eigentliche Füllmedium für die Bestrahlung ersetzt. Der Austausch birgt jedoch das Risiko, dass sich die Lageverhältnisse erneut ändern. Bei der vorangegangenen Verwendung von Kontrastmittel ist nach dem Austausch schwer zu sagen, wie vollständig der Austausch war beziehungsweise ob es irgendwo im Ballonkatheter höher konzentrierte Ansammlungen des Kontrastmittels gibt. Die Auswirkung wäre eine ungleichmäßige Bestrahlung des Tumorbetts, da Reste des Kontrastmittels die Röntgenstrahlung örtlich begrenzt absorbieren. Eine vollständige Abtötung der Tumorzellen ist so nicht gewährleistet.

In anderem Zusammenhang ist aus der WO 99/04856 A1 bereits ein Ballonkatheter bekannt, mit dem die Intensität einer von einer Strahlungsquelle abgegebenen Strahlung, die auf das zu behandelnde Gewebe trifft, eingestellt werden kann. Der bekannte Ballonkatheter weist einen Katheterschaft auf sowie einen ersten äußeren Ballon und einen zweiten, innerhalb des äußeren Ballons angeordneten inneren Ballon. Zwischen dem inneren Ballon und dem äußeren Ballon ist ein Zwischenraum ausgebildet, wobei der Abstand zwischen den Ballonen und damit das Volumen des Zwischenraums, konstant gehalten werden soll. Eine in den Zwischenraum eingebrachte Flüssigkeit hat die Aufgabe, Strahlung zu absorbieren. Auch mit dieser bekannten Lösung kann somit nicht das vorstehend genannte Problem gelöst werden.

Eine weitere Lösung für einen Ballonkatheter mit zwei ineinander liegenden Ballonen ist in der US 2005/0080313 A1 beschrieben. Bei dieser bekannten Lösung sind die beiden Ballone in großen Bereichen miteinander verbunden. Lediglich an bestimmten Stellen sind die beiden Ballone losgelöst voneinander, so dass sich dort ein Spalt ausbildet, in den ein Medium injiziert werden kann. Dadurch soll erreicht werden, dass einzelne Bereiche von Gewebe von einer Bestrahlung ausgeschlossen werden können. Auch bei dieser Lösung ist es nach wie vor nicht möglich, die weiter oben genannten Probleme hinsichtlich der Platzierung des Ballonkatheters zu lösen.

In der EP 0 850 660 B1 ist eine Ballonkathetereinrichtung offenbart, die einen Innenballon und einen Außenballon umfasst, und die zur Einführung in ein Blutgefäß verwendet wird. Beide Ballone sind mit einer Medienzufuhr verbunden, über welche die Ballone aufgeblasen werden können.

In der WO 2008/077073 A2 wird eine Katheterlösung für die Brachytherapie offenbart, mittels derer eine Strahlungsquelle an den Bestrahlungsort gebracht werden kann. Der Katheter weist einen Schaft auf, in welchen die Strahlungsquelle eingeschoben wird. Am distalen Ende des Schafts befindet sich eine Balloneinrichtung, mittels derer durch Aufpumpen das die Strahlungsquelle umgebende Gewebe in eine gewünschte Position gebracht werden kann. Um auch asymmetrische Strahlungsdosisprofile der Strahlungsquelle erzeugen zu können, kann die Balloneinrichtung einen Innenballon und einen Außenballon aufweisen.

Ausgehend vom genannten Stand der Technik liegt der vorliegenden Erfindung die Aufgabe zugrunde, einen Applikator mit Ballonkatheter der eingangs genannten Art derart weiterzubilden, dass eine Kontrolle des Ballonkatheters vor einer Bestrahlung hinsichtlich seiner Lage und Form in einem Patienten auf einfache und dennoch eindeutige Weise sichergestellt ist.

Diese Aufgabe wird erfindungsgemäß gelöst durch den Applikator mit den Merkmalen gemäß dem unabhängigen Patentanspruch 1. Weitere Merkmale und Details der Erfindung ergeben sich aus den Unteransprüchen, der Beschreibung sowie den Zeichnungen.

Der erfindungsgemäße Applikator eignet sich in besonderer Weise zur Erzielung einer Sichtbarkeit von Ballonkathetern in der invasiven Radiotherapie. Die vorliegende Erfindung beruht auf einer Methodik und einer Einrichtung, um einen Ballonkatheter vor einer Bestrahlung hinsichtlich seiner Form und Lage in einem Patienten kontrollieren zu können.

Erfindungsgemäß wird ein Applikator, insbesondere für die invasive Radiotherapie, bereitgestellt, mit einem Ballonkatheter, aufweisend einen Katheterschaft zur Einführung einer Sonde, aufweisend einen ersten äußeren Ballon aus einem flexiblen, nicht dehnbaren Material und einen zweiten, innerhalb des äußeren Ballons angeordneten inneren Ballon aus einem flexiblen, dehnbaren Material, wobei ein Normzustand dadurch definiert ist, dass der innere Ballon ein Füllvolumen V2 = 4/3 x R2³ x Pi mit R2 = Radius des inneren Ballons und der äußere Ballons ein Füllvolumen V1 = 4/3 x R1³ x Pi mit R1 = Radius des äußeren Ballons aufweist, wobei der Radius R2 des inneren Ballons kleiner ist als der Radius R1 des äußeren Ballons, weiterhin aufweisend einen Innenraum des inneren zweiten Ballons, der über einen Verbindungskanal zumindest zeitweilig mit einer Medienzufuhr verbunden ist und weiterhin aufweisend einen Zwischenraum zwischen dem ersten äußeren Ballon und dem zweiten inneren Ballon, der über einen Verbindungskanal mit einem Medienspeicher verbunden ist, wobei der Zwischenraum zwischen dem ersten äußeren Ballon und dem zweiten inneren Ballon, der Verbindungskanal und der Medienspeicher als abgeschlossenes System ausgebildet sind, wobei sich in dem Zwischenraum zwischen dem ersten äußeren Ballon und dem zweiten inneren Ballon zumindest zeitweise ein Medium befindet, mittels dessen die Lage des Ballonkatheters genau geprüft werden kann, wobei in dem abgeschlossenen System ein definiertes Medienvolumen vorgesehen ist, wobei im Normzustand Medium, das sich im Zwischenraum befindet, einen Abstand zwischen dem äußeren Ballon und dem inneren Ballon aufrecht erhält, und wobei das Füllvolumen V2 des inneren Ballons auf das Füllvolumen V1 des äußeren Ballons vergrößert werden kann, so dass der innere Ballon eng an dem äußeren Ballon anliegt und das Medium aus dem Zwischenraum in den Mediumspeicher verdrängt wird.

Ein solcher Applikator besteht aus den Grundkomponenten Ballonkatheter, Medienzufuhr und Medienspeicher.

Der Ballonkatheter weist zunächst einen Katheterschaft zur Einführung einer Sonde auf. Hierüber wird die Einführung einer Sonde, etwa einer Strahlentherapiesonde, ermöglicht.

Weiterhin verfügt der Ballonkatheter zunächst über einen ersten äußeren Ballon (Außenballon). Dieser Außenballon ist erfindungsgemäß aus einem flexiblen, nicht dehnbaren Material gebildet, wobei die Erfindung nicht auf bestimmte Materialien beschränkt ist. Der äußere Ballon weist einen bestimmten Radius R1 auf und kann beispielsweise Polyethylen "PET" oder dergleichen bestehen.

Weiterhin weist der Ballonkatheter einen zweiten, innerhalb des äußeren Ballons angeordneten inneren Ballon (Innenballon) auf. Dieser Innenballon ist erfindungsgemäß aus einem flexiblen, dehnbaren Material gebildet, wobei die Erfindung nicht auf bestimmte Materialien beschränkt ist. Wichtig ist in erster Linie, dass der Innenballon dehnbar ist. Der innere Ballon kann beispielsweise aus einem flexiblen dehnbaren Material wie Silikon; Latex Urethan oder dergleichen bestehen. Der innere Ballon hat im Normzustand einen Radius R2, der kleiner ist als der Radius R1 des Außenballons im Normzustand. Der Normzustand des Innenballons ist durch das Füllvolumen V2= 4/3 x R2³ x Pi definiert, währen der Normzustand des Außenballons durch das Füllvolumen V1 = 4/3 x R1³ x Pi definiert ist.

Das Ballonvolumen V2 des Innenballons kann durch weiteres Zuführen von Füllmedium vergrößert werden, da der Innenballon aus dehnbarem Material besteht. Ist jedoch das Volumen V1 (V1 = 4/3 X R1³ x Pi) des äußeren Ballons erreicht, so kann durch weitere Zugabe von Füllmedium der Innenballon nicht weiter vergrößert werden. Das wird durch den nicht dehnbaren Außenballon verhindert. Weiteres Befüllen des Innenballons erhöht zwar den Druck im Inneren des Ballons und verbessert so die Formstabilität des Ballons, jedoch ohne die Gefahr einer Ballonruptur. Die Formstabilität bietet einen großen Vorteil gegenüber Ballonkathetern aus flexiblen dehnbaren Materialien, da sich die Gegebenheiten während der Bestrahlung nicht ändern. Durch äußere Einflüsse kann der Ballon nicht verformt werden und die optimale Bestrahlung des Tumorbettes ist so gewährleistet.

Erfindungsgemäß ist der Innenraum des inneren zweiten Ballons mit einem Kanal zur Verbindung mit einer Medienzufuhr verbunden. Der Innenballon kann somit über den Kanal mit einem Medium befüllt werden. Dabei ist die Erfindung grundsätzlich nicht auf bestimmte Medientypen beschränkt. Einige vorteilhafte, jedoch nicht ausschließliche Beispiele werden im weitern Verlauf näher erläutert.

Schließlich ist erfindungsgemäß der Zwischenraum zwischen dem ersten äußeren Ballon und dem zweiten inneren Ballon mit einem Kanal zur Verbindung mit einem Medienspeicher verbunden. Der Zwischenraum wird somit durch die äußere Oberfläche des Innenballons und die innere Oberfläche des Außenballons begrenzt. Der Bereich zwischen den beiden Ballonschalen ist über den Verbindungskanal mit dem Mediumspeicher verbunden. In dem Zwischenraum kann sich dann zumindest zeitweise ein bestimmtes Medium befinden, mittels dessen die Lage des Ballonkatheters genau geprüft werden kann. Wie dies im Einzelnen geschehen kann, wird im weiteren Verlauf der Beschreibung, insbesondere im Zusammenhang mit dem erfindungsgemäßen Applikator, in größerem Detail beschrieben, so dass an dieser Stelle auf die entsprechenden Ausführungen Bezug genommen und verwiesen wird.

Vorteilhaft sind der Außenballon und der Innenballon im Bereich des distalen Endes des Katheterschafts vorgesehen. Natürlich könnten die Ballone auch an anderen Stellen des Katheterschafts vorgesehen beziehungsweise angeordnet sein.

Vorzugsweise ist/sind der Außenballon und/oder der Innenballon sphärisch, zigarrenförmig oder elliptisch ausgebildet, wobei die Erfindung nicht auf bestimmte Konturen beschränkt ist. Als besonders bevorzugt ist eine sphärische Ausgestaltung anzusehen.

In weiterer Ausgestaltung kann im Katheterschaft im Bereich des Ballonzentrums des Innenballons und/oder Außenballons vorteilhaft eine Positioniereinrichtung zur Positionierung einer Sonde vorgesehen sein. Hierbei kann es sich beispielsweise um einen Anschlag, beispielsweise einen mechanischen Anschlag, handeln. Eine Sonde kann dann bis zu dieser Positioniereinrichtung in den Katheterschaft eingeführt und über die Positioniereinrichtung genau positioniert werden. Dadurch wird erreicht, dass die Sonde die Strahlung in der gewünschten Menge und Richtung an der richtigen Stelle abgibt. Vorzugsweise befindet sich die Positioniereinrichtung im Ballonmittelpunkt des Außenballons und/oder Innenballons.

Erfindungsgemäß ist vorgesehen, dass der Zwischenraum zwischen dem ersten äußeren Ballon und dem zweiten inneren Ballon, der Verbindungskanal und der Medienspeicher als abgeschlossenes System ausgebildet sind. Das bedeutet, dass dem System von außen weder Medium zugeführt wird, noch dass Medium aus diesem System abgeführt wird.

In dem abgeschlossenen System ist ein definiertes Medienvolumen vorgesehen.

Vorteilhaft kann der Medienspeicher als abgeschlossener, nicht dehnbarer Behälter oder als Behälter aus einem flexiblen dehnbaren Material ausgebildet sein. Natürlich ist die Erfindung nicht auf diese beiden Ausgestaltungsformen beschränkt. Der Mediumspeicher kann in verschiedenen Umsetzungsversionen ausgeführt sein. Bei einem gasförmigen Medium beispielsweise ist die Verwendung eines abgeschlossenen nicht dehnbaren Behälters sinnvoll, da sich Gase sehr gut komprimieren lassen. Es muss kein Gas abgelassen oder zugeführt werden, wodurch sich Risiken infolge von Fehlbedienung für den Patienten minimieren. Der Mediumspeicher kann aber auch aus einem flexiblen dehnbaren Material bestehen, so dass dieser das aus dem Ballonzwischenbereich verdrängte Volumen aufnehmen kann. Außerdem kann anstatt eines abgeschlossenen Behälters auch ein System temporär angeschlossen werden, welches das verdrängte Medium aufnimmt beziehungsweise wieder definiert zuführt. Im einfachsten Fall ist es eine Injektionsspritze oder ähnliches.

Vorteilhaft kann deshalb auch vorgesehen sein, dass der Medienspeicher und/oder die Medienzufuhr lösbar mit dem Verbindungskanal verbunden ist/sind.

Nachfolgend werden einige vorteilhafte, jedoch nicht ausschließliche Beispiele geeigneter Medien beschrieben. Beispielsweise kann das im Medienspeicher befindliche Medium eine Flüssigkeit oder ein Gas, insbesondere Luft, eine Wasserdispersion oder ein flüssiges oder gasförmiges Kontrastmittel, sein. Beispielsweise kann das über die Medienzufuhr zugeführte Medium eine Flüssigkeit oder ein Gas, insbesondere Luft, eine wässrige Lösung, eine Lösung aus Kontrastmittel oder eine Kochsalzlösung, sein.

Vorteilhaft ist vorgesehen, dass in dem Katheterschaft zumindest zeitweilig eine Sonde eingeführt ist. Die Sonde befindet sich insbesondere während des bestimmungsgemäßen Gebrauchs des Applikators innerhalb des Katheterschafts.

Die Erfindung, wie sie hinsichtlich des Ballonkatheters sowie des Applikators beschrieben ist, beinhaltet des Weiteren auch folgende Methodik:
Ein zunächst zusammengefalteter Ballonkatheter wird in das zu behandelnde Gewebe gelegt. Zu diesem Zeitpunkt befindet sich noch kein Medium im Innenballon, so dass der Ballonkatheter seine minimale Größe annimmt. Der Ballonkatheter wird im zu behandelnden Gewebe positioniert und eine Sonde in den Katheterschaft, der einen Kanal bildet, definiert eingeführt. Der Ballonkatheter kann aber auch direkt in das zu behandelnde Gewebe eingelegt werden.

Anschließend wird der Innenballon über den Verbindungskanalanal aus der Medienzufuhr bis zu einem Normzustand V2 gefüllt. Die Befüllung kann auf unterschiedliche Weise erfolgen, beispielsweise manuell mit einer Injektionsspritze, automatisiert mit einer Pumpanordnung, oder dergleichen. Das Füllmedium, das sich im Zwischenbereich zwischen Innenballon und Außenballon befindet, wird so aus dem Bereich V2 in den Zwischenbereich verdrängt, wo es zunächst den Abstand zwischen Außenballon und Innenballon aufrecht hält.

In diesem Zustand kann mit verschiedenen bildgebenden Verfahren die Lageposition und Form des Ballonkatheters im Patienten überprüft und gegebenenfalls korrigiert werden. Die Voraussetzung dabei ist das Vorhandensein eines Mediums im Ballonzwischenraum, welches sich ausreichend vom umgebenden Gewebe unterscheidet, so dass es für die Bildgebung genutzt werden kann.

Liegt der Ballonkatheter korrekt, wird zusätzliches Medium über den Verbindungskanal aus der Medienzufuhr in den Innenballon eingeführt, bis sich der Innenballon soweit ausgedehnt hat, dass er eng am Außenballon anliegt. Dadurch wird das Medium aus dem Ballonzwischenraum in den Mediumspeicher verdrängt. In diesem Zustand kann die Bestrahlung des Tumorbettes durchgeführt werden.

Bei dieser Methode wird das Kontrastmedium für die Sichtbarkeit ohne Druckverminderung aus dem Bestrahlungsfeld entfernt. Durch die Aufrechterhaltung des Drucks ergibt sich der Vorteil, dass sich die Lageverhältnisse zwischen Sonde, Ballonkatheter und zu behandelndem Gewebe nicht ändern. Aus diesem Grund ist es vorteilhaft, den Ballonzwischenraum, den Verbindungskanal und den Mediumspeicher als abgeschlossenes System vorzusehen. Ein weiterer Vorteil ergibt sich durch die nur temporäre Anwesenheit eines Kontrastmittels im Bestrahlungsfeld. Dadurch kommt es nicht zu einer ungewollten Strahlenabsorption während der eigentlichen Bestrahlung, wodurch die Dosisleistung gesenkt oder die Bestrahlungszeit minimiert werden kann.

Nach der Bestrahlung wird der Innenballon über den Verbindungskanal komplett entleert, wodurch sich auch der Gesamtdurchmesser des Ballonkatheters minimiert. Nach der Entfernung der Sonde kann der Ballonkatheter wieder entfernt werden.

Im Fall einer fraktionierten Bestrahlung verbleibt der Ballonkatheter üblicherweise im Patienten und es wird eine weitere Bestrahlung zu einem späteren Zeitpunkt durchgeführt. Vor jeder Bestrahlung werden erneut die Lageverhältnisse von Sonde, Ballonkatheter und zu behandelndem Gewebe überprüft. Hierfür kann der oben beschriebene Vorgang wie folgt rückgängig gemacht werden. Das Füllmedium wird über den Verbindungskanal aus dem Innenballon zum Teil abgelassen, bis der Innenballon seinen Normzustand V2 erreicht hat. Dabei strömt Medium aufgrund der sich ändernden Druckverhältnisse aus dem Mediumspeicher in den Ballonzwischenraum. Das Medium kann aber auch erneut aktiv zugeführt werden. Jetzt können die Lageverhältnisse wiederholt mit bildgebenen Verfahren überprüft und gegebenenfalls korrigiert werden. Anschließend wird, wie bereits beschrieben, die Behandlung fortgesetzt.

Die Erfindung wird nun anhand von Ausführungsbeispielen unter Bezugnahme auf die beiliegenden Zeichnungen näher erläutert. Es zeigen
- Figur 1: den Grundaufbau eines erfindungsgemäßen Applikators;
- Figur 2: den Applikator bei Hinführung zu dem zu behandelnden Gewebe;
- Figur 3: den Applikator im Zustand während einer Lageübeprüfung; und
- Figur 4: den Applikator im Zustand während eines Bestrahlungsvorgangs.

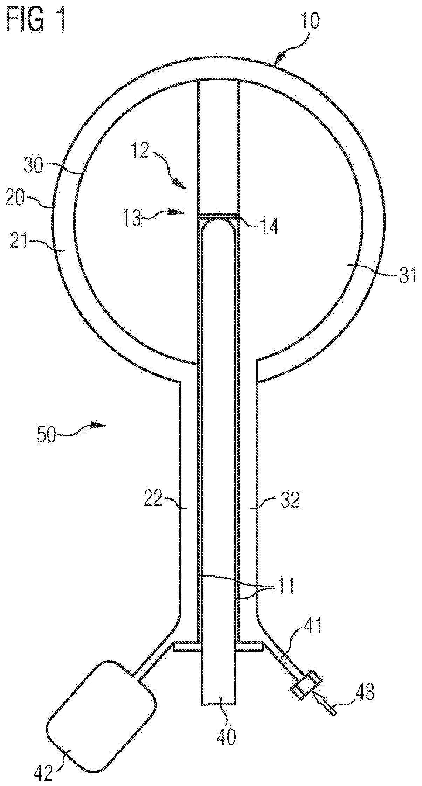

In den Figuren 1 bis 4 ist ein Applikator 50 mit einem Ballonkatheter 10 dargestellt, der für die invasive Radiotherapie eingesetzt werden kann.

Figur 1 zeigt zunächst den Grundaufbau des Applikators 50. Dieser verfügt zunächst über einen Ballonkatheter 10, bestehend aus einem Katheterschaft 11. Im Bereich des distalen Endes 12 des Katheterschafts 11 ist eine Positioniereinrichtung 14 in Form eines Anschlags vorgesehen, um eine in den Katheterschaft 11 eingeführte Sonde 40 zu positionieren.

Im Bereich des distalen Endes 12 des Katheterschafts 11 sind weiterhin ein Außenballon 20 und ein innerhalb des Außenballons 20 befindlicher Innenballon 30 vorgesehen. Der Innenballon 30 begrenzt einen Innenraum 31, der über einen Verbindungskanal 32 mit einer Medienzufuhr 41 verbunden ist, über die ein Medium 43 zugeführt, aber auch wieder abgeführt werden kann. Durch die Wandungen des Innenballons 30 und des Außenballons 20 wird ein Zwischenraum 21 begrenzt, der über einen Verbindungskanal 22 mit einem Mediumspeicher 42 verbunden ist.

Wie in Figur 1 weiterhin dargestellt ist, befindet sich die Positioniereinrichtung 14 im Bereich des Ballonzentrums 13 sowohl vom Innenballon 30 als auch vom Außenballon 20.

Der in den Figuren 1 bis 4 dargestellte Applikator 50 soll dazu dienen, ein Tumorbett zu bestrahlen.

Das in den Figuren dargestellte Ausführungsbeispiel beschreibt sowohl eine Methodik als auch ein Gerät, um den Ballonkatheter 10 vor der Bestrahlung hinsichtlich seiner Form und Lage im Patienten zu kontrollieren.

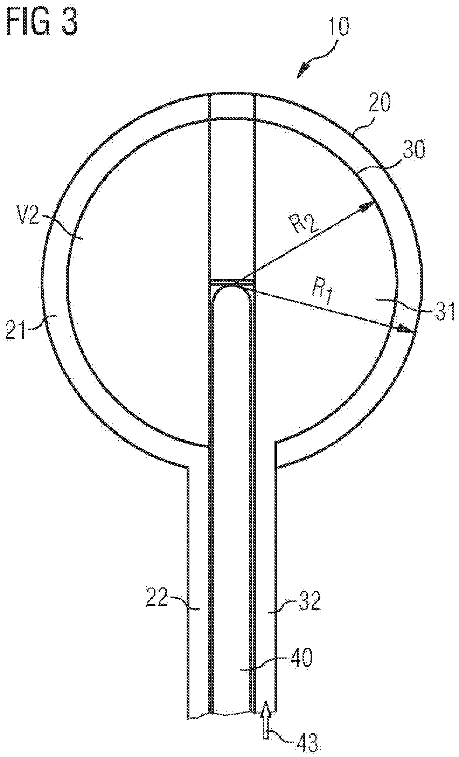

Figur 1 zeigt den schematischen Aufbau eines Ballonkatheters 10 in sphärischer Form. Die Form des Ballons kann aber auch zigarrenförmig, elliptisch oder dergleichen sein. Der Ballonkatheter 10 besteht aus dem Außenballon 20 und dem, insbesondere kleineren, Innenballon 30. Der Außenballon mit dem Radius R1, wie in Figur 3 dargestellt ist, besteht aus einem flexiblen, nicht dehnbaren Material wie beispielsweise Polyethylen "PET". Der Innenballon 30 hingegen besteht aus einem flexiblen, dehnbaren Material wie beispielsweise Silikon; Latex, Urethan oder dergleichen.

Der Innenballon 30 hat im Normzustand den Radius R2, wie in Figur 3 dargestellt ist. Der Normzustand ist durch das Füllvolumen V2= 4/3 x R2³ x Pi definiert.

Der Katheterschaft 11 ermöglicht die Einführung einer Sonde 40 bis in den Bereich des Ballonzentrums 13 der Ballone 20, 30. Im Ballonzentrum 13 hilft ein mechanischer Anschlag 14 für die genaue Positionierung der Sonde 40. Dieser Anschlag 14 ist nicht zwingend erforderlich.

Der Zwischenraum 21 zwischen den beiden Ballonschalen des Außenballons 20 und des Innenballons 30 ist über den Verbindungskanal 22 mit dem Mediumspeicher 42 verbunden. In diesem abgeschlossenen System befindet sich ein definiertes Volumen eines Mediums in Form von Luft, einer Wasserdispersion oder einer Kontrastmittelflüssigkeit.

Der Innenballon 30 kann über den Verbindungskanal 32 mit einem Medium 43 befüllt werden. Das Medium 43 kann Luft, Wasser oder eine Lösung aus Kontrastmittel sein.

Das Ballonvolumen V2 des Innenballons 30 kann durch weiteres Zuführen von Füllmedium vergrößert werden, da der Innenballon 30 aus dehnbarem Material besteht. Ist jedoch das Volumen V1 (V1 = 4/3 X R1³ x Pi) des Außenballons 20 erreicht, so kann durch weitere Zugabe von Füllmedium der Innenballon 30 nicht weiter vergrößert werden. Das wird durch den nicht dehnbaren Außenballon 20 verhindert. Ein weiteres Befüllen des Innenballons erhöht zwar den Druck im Innenraum 31 des Innenballons 30, wodurch die Formstabilität des Innenballons verbessert wird, jedoch ohne die Gefahr einer Ballonruptur. Die Formstabilität bietet einen großen Vorteil gegenüber Ballonkathetern aus flexiblen dehnbaren Materialien, da sich die Gegebenheiten während der Bestrahlung nicht ändern. Durch äußere Einflüsse kann der Ballon nicht verformt werden und die optimale Bestrahlung des Tumorbettes ist so gewährleistet.

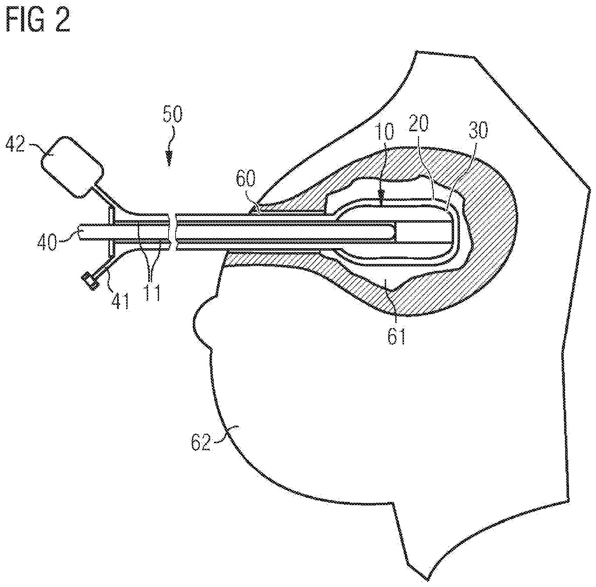

Wie in Figur 2 dargestellt ist, wird der zunächst zusammengefaltete Ballonkatheter 10 über einen Biopsiekanal 60 in das Tumorbett 61 gelegt. Zu diesem Zeitpunkt befindet sich noch kein Medium im Innenballon 30, so dass der Ballonkatheter 10 seine minimale Größe annimmt und in den Biopsiekanal 60 eingeführt werden kann. Der Ballonkatheter 10 wird im Tumorbett 61 positioniert und die Sonde 40 in den Katheterschaft 11 definiert eingeführt. Figur 2 zeigt das Prinzip am Beispiel einer Brust 62. Der Ballonkatheter 10 kann aber auch direkt über den chirurgischen Zugang die Tumorextraktion in das Tumorbett 61 gelegt werden.

Anschließend wird der Innenballon 30 über den Verbindungskanal 32 bis zum Normzustand V2 gefüllt, was in Figur 3 dargestellt ist. Die Befüllung kann beispielsweise manuell mit einer Injektionsspritze, oder automatisiert mit einer Pumpanordnung erfolgen. Das Füllmedium des Zwischenraums 21 zwischen den beiden Ballonen 20, 30 wird so aus dem Bereich V2 in den Zwischenbereich verdrängt, wo es den Abstand zwischen innerer und äußerem Ballon 20, 30 aufrecht hält und gewährleistet.

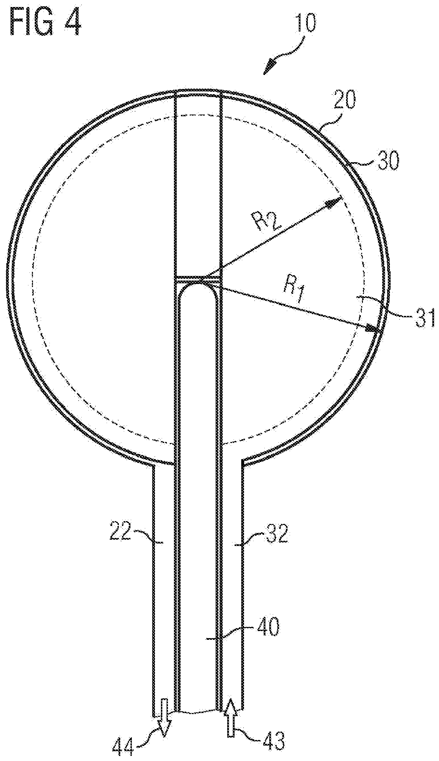

In diesem Zustand kann mit verschiedenen bildgebenden Verfahren die Lageposition und Form des Ballonkatheters 10 im Patienten überprüft und gegebenenfalls korrigiert werden. Die Voraussetzung hier ist das Vorhandensein eines Mediums im Ballonzwischenraum 21, welches sich ausreichend vom umgebenden Gewebe unterscheidet, so dass es für die Bildgebung genutzt werden kann. Liegt der Ballonkatheter 10 korrekt, wird weiter Medium 43 über den Verbindungskanal 32 in den Innenraum 31 des Innenballons 30 zugegeben, bis sich der Innenballon 30 soweit ausgedehnt hat, dass er eng am Außenballon 20 anliegt, wie in Figur 4 dargestellt ist.

Die ursprüngliche Größe des Innenballons 30 ist in Figur 4 gestrichelt dargestellt. Dadurch wird das Medium aus dem Ballonzwischenraum 21 in den Mediumspeicher 42 verdrängt, was durch Pfeil 44 dargestellt ist. In diesem Zustand kann die Bestrahlung des Tumorbettes 61 durchgeführt werden. Bei dieser Methode wird das Kontrastmedium für die Sichtbarkeit ohne Druckverminderung aus dem Bestrahlungsfeld entfernt. Durch die Aufrechterhaltung des Drucks ergibt sich der Vorteil, dass sich die Lageverhältnisse zwischen Sonde 40, Ballonkatheter 10 und Tumorbett 61 nicht ändern. Ein weiterer Vorteil ergibt sich durch die nur temporäre Anwesenheit eines Kontrastmittels im Bestrahlungsfeld. Dadurch kommt es nicht zu einer ungewollten Röntgenstrahlenabsorption während der eigentlichen Bestrahlung, wodurch die Dosisleistung gesenkt oder die Bestrahlungszeit minimiert werden kann.

Nach der Bestrahlung wird der Innenballon 30 über den Verbindungskanal 32 komplett entleert, wodurch sich auch der Gesamtdurchmesser des Ballonkatheters 10 minimiert. Nach der Entfernung der Sonde 40 kann der Ballonkatheter 10 wieder durch den Biopsiekanal 60 entfernt werden.

### Bezugszeichenliste

- 10: Ballonkatheter
- 11: Katheterschaft
- 12: Distales Ende des Katheterschafts
- 13: Ballonzentrum
- 14: Positioniereinrichtung (Anschlag)

- 20: Äußerer Ballon (Außenballon)
- 21: Zwischenraum
- 22: Verbindungskanal

- 30: Innerer Ballon (Innenballon)
- 31: Innenraum
- 32: Verbindungskanal

- 40: Sonde
- 41: Medienzufuhr
- 42: Mediumspeicher
- 43: Zugeführtes Medium
- 44: Verdrängungsrichtung des Mediums aus dem Zwischenraum

- 50: Applikator

- 60: Biopsiekanal
- 61: Tumorbett
- 62: Brust

- R1: Radius Normzustand Außenballon
- R2: Radius Normzustand Innenballon
- V2: Volumen Normzustand Innenballon

## Patentansprüche

1. Applikator (50), insbesondere für die invasive Radiotherapie, mit einem Ballonkatheter (10), aufweisend einen Katheterschaft (11) zur Einführung einer Sonde (40), aufweisend einen ersten äußeren Ballon (20) aus einem flexiblen, nicht dehnbaren Material und einen zweiten, innerhalb des äußeren Ballons (20) angeordneten inneren Ballon (30) aus einem flexiblen, dehnbaren Material, wobei ein Normzustand dadurch definiert ist, dass der innere Ballon (30) ein Füllvolumen V2 = 4/3 x R2³ x Pi mit R2 = Radius des inneren Ballons (30) und der äußere Ballons (20) ein Füllvolumen V1 = 4/3 x R1³ xPi mit R1 = Radius des äußeren Ballons aufweist, wobei der Radius R2 des inneren Ballons (30) kleiner ist als der Radius R1 des äußeren Ballons (20), weiterhin aufweisend einen Innenraum (31) des inneren zweiten Ballons (30), der über einen Verbindungskanal (32) zumindest zeitweilig mit einer Medienzufuhr (41) verbunden ist und weiterhin aufweisend einen Zwischenraum (21) zwischen dem ersten äußeren Ballon (20) und dem zweiten inneren Ballon (30), der über einen Verbindungskanal (22) mit einem Medienspeicher (42) verbunden ist, wobei der Zwischenraum (21) zwischen dem ersten äußeren Ballon (20) und dem zweiten inneren Ballon (30), der Verbindungskanal (22) und der Medienspeicher (42) als abgeschlossenes System ausgebildet sind, wobei sich in dem Zwischenraum (21) zwischen dem ersten äußeren Ballon (20) und dem zweiten inneren Ballon (30) zumindest zeitweise ein Medium befindet, mittels dessen die Lage des Ballonkatheters genau geprüft werden kann, wobei in dem abgeschlossenen System ein definiertes Medienvolumen vorgesehen ist, wobei im Normzustand Medium, das sich im Zwischenraum (21) befindet, einen Abstand zwischen dem äußeren Ballon (20) und dem inneren Ballon (30) aufrecht erhält, und wobei das Füllvolumen V2 des inneren Ballons (30) auf das Füllvolumen V1 des äußeren Ballons (20) vergrößert werden kann, so dass der innere Ballon (30) eng an dem äußeren Ballon (20) anliegt und das Medium aus dem Zwischenraum (21) in den Mediumspeicher (42) verdrängt wird.

2. Applikator nach Anspruch 1, **dadurch gekennzeichnet, dass** der Medienspeicher (42) als abgeschlossener, nicht dehnbarer Behälter oder als Behälter aus einem flexiblen dehnbaren Material ausgebildet ist

3. Applikator nach Anspruch 1 oder 2, **dadurch gekennzeichnet, dass** der Medienspeicher (42) und/oder die Medienzufuhr (41) lösbar mit dem Verbindungskanal verbunden ist/sind.

4. Applikator nach einem der Ansprüche 1 bis 3, **dadurch gekennzeichnet, dass** das im Medienspeicher (42) befindliche Medium eine Flüssigkeit oder ein Gas, insbesondere Luft, eine Wasserdispersion oder ein flüssiges oder gasförmiges Kontrastmittel, ist.

5. Applikator nach einem der Ansprüche 1 bis 4, **dadurch gekennzeichnet, dass** das über die Medienzufuhr (41) zugeführte Medium eine Flüssigkeit oder ein Gas, insbesondere Luft, eine wässrige Lösung, eine Lösung aus Kontrastmittel oder eine Kochsalzlösung, ist.

6. Applikator nach einem der Ansprüche 1 bis 5, **dadurch gekennzeichnet, dass** in dem Katheterschaft (11) zumindest zeitweilig eine Sonde (40) eingeführt ist.

7. Applikator nach einem der Ansprüche 1 bis 6, **dadurch gekennzeichnet, dass** der Außenballon (20) und der Innenballon (30) im Bereich des distalen Endes (12) des Katheterschafts (11) vorgesehen sind.

8. Applikator nach einem der Ansprüche 1 bis 7, **dadurch gekennzeichnet, dass** der Außenballon (20) und/oder der Innenballon (30) sphärisch, zigarrenförmig oder elliptisch ausgebildet ist/sind.

9. Applikator nach einem der Ansprüche 1 bis 8, **dadurch gekennzeichnet, dass** im Katheterschaft (11) im Bereich des Ballonzentrums (13) des Innenballons (30) und/oder Außenballons (20) eine Positioniereinrichtung (14) zur Positionierung einer Sonde (40) vorgesehen ist.

## Claims

1. Applicator (50), in particular for invasive radiotherapy, with a balloon catheter (10), having a catheter shaft (11) for introduction of a probe (40), having a first, outer balloon (20) made of a flexible, non-extensible material, and a second, inner balloon (30) arranged inside the outer balloon (20) and made of a flexible, extensible material, wherein a standard state is defined as being that the inner balloon (30) has a filling volume V2 = 4/3 × R2³ × Pi with R2 = radius of the inner balloon (30), and the outer balloon (20) has a filling volume V1 = 4/3 × R1³ × Pi with R1 = radius of the outer balloon, wherein the radius R2 of the inner balloon (30) is smaller than the radius R1 of the outer balloon (20), furthermore having an interior (31) of the inner, second balloon (30), which interior (31) is at least temporarily connected to a media inlet (41) via a connection channel (32), and furthermore having an intermediate space (21) between the first, outer balloon (20) and the second, inner balloon (30), which intermediate space (21) is connected to a media reservoir (42) via a connection channel (22), wherein the intermediate space (21) between the first, outer balloon (20) and the second, inner balloon (30), the connection channel (22) and the media reservoir (42) are configured as a closed system, wherein a medium by means of which the position of the balloon catheter can be examined precisely is located at least temporarily in the intermediate space (21) between the first, outer balloon (20) and the second, inner balloon (30), wherein a defined volume of media is provided in the closed system, wherein, in the standard state, medium located in the intermediate space (21) maintains a spacing between the outer balloon (20) and the inner balloon (30), and wherein the filling volume V2 of the inner balloon (30) can be increased to the filling volume V1 of the outer balloon (20), such that the inner balloon (30) bears closely on the outer balloon (20) and the medium is displaced from the intermediate space (21) into the media reservoir (42) .

2. Applicator according to Claim 1, **characterized in that** the media reservoir (42) is configured as a closed, non-extensible container or as a container made of a flexible, extensible material.

3. Applicator according to Claim 1 or 2, **characterized in that** the media reservoir (42) and/or the media inlet (41) are/is connected releasably to the connection channel.

4. Applicator according to one of Claims 1 to 3, **characterized in that** the medium located in the media reservoir (42) is a liquid or a gas, in particular air, a dispersion in water, or a liquid or gaseous contrast agent.

5. Applicator according to one of Claims 1 to 4, **characterized in that** the medium supplied via the media inlet (41) is a liquid or a gas, in particular air, an aqueous solution, a solution of contrast agent or a saline solution.

6. Applicator according to one of Claims 1 to 5, **characterized in that** a probe (40) is introduced at least temporarily into the catheter shaft (11).

7. Applicator according to one of Claims 1 to 6, **characterized in that** the outer balloon (20) and the inner balloon (30) are provided in the region of the distal end (12) of the catheter shaft (11).

8. Applicator according to one of Claims 1 to 7, **characterized in that** the outer balloon (20) and/or the inner balloon (30) are/is of a spherical, cigar-shaped or elliptical configuration.

9. Applicator according to one of Claims 1 to 8, **characterized in that** a positioning device (14) for positioning a probe (40) is provided in the catheter shaft (11) in the region of the balloon centre (13) of the inner balloon (30) and/or outer balloon (20).

## Revendications

1. Applicateur (50), en particulier pour la radiothérapie invasive, avec un cathéter à ballonnet (10), comprenant une tige de cathéter (11) pour introduction d'une sonde (40), comportant un premier ballonnet extérieur (20) fait d'un matériau flexible non élastique et un deuxième ballonnet intérieur (30) agencé à l'intérieur du ballonnet extérieur (20), fait d'un matériau flexible élastique, dans lequel un état normal est défini en ce que le ballonnet intérieur (30) possède un volume de remplissage V2 = 4/3 × R2³ × Pi avec R2 = rayon du ballonnet intérieur (30) et le ballonnet extérieur (20) possède un volume de remplissage V1 = 4/3 × R1³ × Pi avec R1 = rayon du ballonnet extérieur, dans lequel le rayon R2 du ballonnet intérieur (30) est inférieur au rayon R1 du ballonnet extérieur (20), comportant en outre un espace intérieur (31) du deuxième ballonnet intérieur (30), lequel est relié au moins temporairement à une alimentation (41) par le biais d'un canal de raccordement (32) et comportant en outre un espace intermédiaire (21) entre le premier ballonnet extérieur (20) et le deuxième ballonnet intérieur (30), lequel est relié à un réservoir (42) par le biais d'un canal de raccordement (22), dans lequel l'espace intermédiaire (21) entre le premier ballonnet extérieur (20) et le deuxième ballonnet intérieur (30), le canal de raccordement (22) et le réservoir (42) sont réalisés comme un système fermé, dans lequel un milieu se trouve au moins temporairement dans l'espace intermédiaire (21) entre le premier ballonnet extérieur (20) et le deuxième ballonnet intérieur (30), au moyen duquel la position du cathéter à ballonnet peut être vérifiée avec exactitude, dans lequel un volume de milieu défini est prévu dans le système fermé, dans lequel, à l'état normal, le milieu se trouvant dans l'espace intermédiaire (21) assure un écart entre le premier ballonnet extérieur (20) et le deuxième ballonnet intérieur (30), et dans lequel le volume de remplissage V2 du ballonnet intérieur (30) peut être agrandi au volume de remplissage V1 du ballonnet extérieur (20), de sorte que le ballonnet intérieur (30) colle étroitement au ballonnet extérieur (20) et le milieu est refoulé dans le réservoir (42) depuis l'espace intermédiaire (21).

2. Applicateur selon la revendication 1, **caractérisé en ce que** le réservoir (42) est réalisé comme récipient fermé non-élastique ou comme récipient fait d'un matériau flexible élastique.

3. Applicateur selon la revendication 1 ou 2, **caractérisé en ce que** le réservoir (42) et/ou l'alimentation (41) sont reliés de manière détachable au canal de raccordement.

4. Applicateur selon l'une des revendications 1 à 3, **caractérisé en ce que** le milieu se trouvant dans le réservoir (42) est un liquide ou un gaz, en particulier de l'air, une dispersion aqueuse ou un agent de contraste liquide ou gazeux.

5. Applicateur selon l'une des revendications 1 à 4, **caractérisé en ce que** le milieu apporté par l'alimentation (41) est un liquide ou un gaz, en particulier de l'air, une solution aqueuse, une solution d'agent de contraste ou une solution saline.

6. Applicateur selon l'une des revendications 1 à 5, **caractérisé en ce qu'**une sonde (40) est introduite au moins temporairement dans la tige de cathéter (11).

7. Applicateur selon l'une des revendications 1 à 6, **caractérisé en ce que** le ballonnet extérieur (20) et le ballonnet intérieur (30) sont prévus dans la zone de l'extrémité distale (12) de la tige de cathéter (11).

8. Applicateur selon l'une des revendications 1 à 7, **caractérisé en ce que** le ballonnet extérieur (20) et/ou le ballonnet intérieur (30) sont réalisés de manière sphérique, en forme de cigare ou elliptique.

9. Applicateur selon l'une des revendications 1 à 8, **caractérisé en ce qu'**un moyen de positionnement (14) est prévu dans la tige de cathéter (11) dans la zone du centre du ballonnet (13) du ballonnet extérieur (20) et/ou du ballonnet intérieur (30) pour positionner une sonde (40).
